# EUROPEAN PATENT APPLICATION

(11) **EP 4 014 866 A1**
(43) Date of publication of application: **22.06.2022**
(21) Application number: 20852250.8
(22) Date of filing: 14.05.2020
(51) Int. Cl.: A61B 5/291, A61B 5/31

(54) **BIOELECTRODE**

(30) Priority: 14.08.2019 JP 2019148721
(71) Applicant: NOK Corporation, Tokyo 105-8585 (JP)
(72) Inventor: OKADA, Goki, Fujisawa-shi, Kanagawa 251-0042 (JP); HAYASHI, Takahiro, Fujisawa-shi, Kanagawa 251-0042 (JP); FUNAHASHI, Akira, Fujisawa-shi, Kanagawa 251-0042 (JP); KUBO, Masayuki, Fujisawa-shi, Kanagawa 251-0042 (JP)
(74) Representative: Global IP Europe Patentanwaltskanzlei
(86) International application number: PCT/JP2020/019215
(87) International publication number: WO 2021/029118

(57) **Abstract**

There is provided a biological electrode capable of realizing good contact with a skin of a subject for detection. The biological electrode includes an electrode member 2, a support shaft member 5 for supporting the electrode member 2, a frame member 6 for slidably holding the support shaft member 5 in an axial direction thereof, and an elastic member 7 for biasing the electrode member 2 toward the outside in the axial direction of the support shaft member 5. The support shaft member 5 and the frame member 6 have a rotation guide mechanism 10 that converts a part of a pressing force when the electrode member 2 is pushed in the axial direction of the support shaft member 5 into a rotation force in which the support shaft member 5 is a rotation axis thereof, the electrode member 2 is pushed in the axial direction while rotating in the peripheral direction of the support shaft member 5 by being pressed in the axial direction, and, when the pressing force for pushing the electrode member 2 is released, receiving the bias from the elastic member 7, the electrode member 2 returns while rotating in the reverse direction to that when it is pushed in.

## Description

### Technical Field

The present invention relates to a biological electrode and, more particularly, to a biological electrode capable of realizing good contact with a skin of the subject for detection.

### Background Art

In order to diagnose a health condition of the subject for detection in a medical facility or the like, a biological electrode is disposed on a body (human body) of the subject for detection and various electric signals are detected. For example, electroencephalogram measurement is performed by disposing an electrode on a scalp of the subject. In recent years, electroencephalogram measurement and the like in daily life as well as at the time of examination are sometimes performed, and a device having a biological electrode capable of stably measuring for a long time has been demanded.

Conventionally, as a biological electrode used for electroencephalogram measurement or the like, a cup electrode in which a thin plate made of a highly conductive metal such as silver or gold is processed into a cup shape has been used. However, since these metal biological electrodes have poor adhesion to the skin and insufficient detection of an electric signal from the skin, it is necessary to apply gel, cream, paste, or the like to the skin. In this case, since the applied material remains on hair or the like and causes discomfort, aftertreatment such as hair washing has been required.

In recent years, there have been proposed an electrode using a probe made of metal (Patent Document 1) or a conductive rubber (Patent Document 2) as an electrode that does not require application of cream, paste, or the like. Further, there has also been proposed a biological electrode in which a flexible or stretchable protrusion is formed on a chip portion which receives an electric signal in the biological electrode (Patent Document 3). Patent Document 3 also proposes a biological electrode in which a support portion supporting a chip portion is configured to be rotatable about a rotation axis perpendicular to a contact surface with a measurement target. Further, there has been proposed a biological electrode (Patent Document 4) in which a plurality of electrode terminals are arranged to a rotary part rotatably provided on a brace mountable on a living body.

### Citation List

### Patent Documents

[Patent Document 1] JP-A-2013-248306
[Patent Document 2] WO-A-2018/230445
[Patent Document 3] JP-A-2011-120866
[Patent Document 4] JP-A-2012-110535

### Summary of the Invention

### Problem to be solved by the Invention

When electroencephalogram measurement with a biological electrode is performed, in order to stably contact the biological electrode with a scalp, it is necessary to push hair aside and directly contact the biological electrode with the scalp. However, in the biological electrodes such as in Patent Documents 1 and 2 in which it is not necessary to apply cream, paste, or the like, the hair is easily caught between the scalp and the biological electrode, and stable contact with the scalp is difficult.

In addition, multi-point contact is required for stable measurement of the biological electrode, and for example, a brush-like electrode having a plurality of protrusions at its tip may be used. However, even with such a brush-like electrode, it is difficult to obtain a good contact state because a movement of pushing hair aside cannot be obtained when mounting. In addition, even for a biological electrode in which a tip portion is configured to be rotatable such as in Patent Documents 3 and 4, when the electrode is simply pressed against the hair and mounted, a movement of positively pushing hair aside cannot be sufficiently obtained, and it is difficult to obtain a good contact state.

In view of the above problems, according to the present invention, there is provided a biological electrode capable of realizing good contact with a skin of a subject for detection. In particular, there is provided a biological electrode which is suitable for electroencephalogram measurement and which is capable of positively pushing hair aside when mounting to realize good contact with the scalp of the subject for detection.

### Means for Solving the Problem

In order to solve the above problems, the present invention provides the following biological electrodes.
[1] A biological electrode including,
   an electrode member in contact with a body of a subject for detection,
   a conductive support shaft member for supporting the electrode member,
   a frame member for slidably holding the support shaft member in an axial direction thereof, and
   an elastic member for biasing the electrode member toward the outside in the axial direction of the support shaft member,
   wherein the electrode member includes a plate-shaped electrode portion and a plurality of electrode protrusions provided so as to protrude in a brush shape from the electrode portion, and
   wherein the support shaft member and the frame member have a rotation guide mechanism that converts a part of a pressing force for pushing the electrode member in the axial direction of the support shaft member into a rotational force in which the support shaft member is a rotation axis thereof.
[2] The biological electrode according to [1], wherein the rotation guide mechanism includes a spiral groove portion provided on an outer peripheral surface of the support shaft member, and a fitting protrusion fitted to the groove portion and provided on an inner peripheral surface of the frame member for holding the support shaft member.
[3] The biological electrode according to [1] or [2], wherein the electrode protrusion is made of conductive rubber.
[4] The biological electrode according to any one of [1] to [3], wherein the elastic member is a coil spring provided so as to wind the outer periphery of the support shaft member.
[5] The biological electrode according to any one of [1] to [4], which is used for electroencephalogram measurement of the subject.
[6] The biological electrode according to [5], wherein the frame member is a part of a head mounting member for mounting on a head of the subject.
[7] The biological electrode according to any one of [1] to [6], further including a terminal portion for transmitting and receiving an electric signal from the electrode member at an end of the support shaft member opposite to the side on which the electrode member is disposed.

### Effects of the Invention

In the biological electrode of the present invention, the electrode member is pressed in an axial direction thereof against the biasing force of the elastic member, whereby the electrode member is pushed in the axial direction while being rotated in the peripheral direction of the support shaft member by the rotation guide mechanism. In addition, when the pressing force pushing the electrode member is released or is smaller than the biasing force of the elastic member, the electrode member is received the bias from the elastic member and pushed out in the axial direction while rotating in the opposite direction to the pushing, and returns. Such a biological electrode can realize good contact with the skin of the subject. In particular, such a biological electrode is suitable for electroencephalogram measurement, and it is possible to positively push hair aside when mounting to realize extremely good contact with the scalp of the subject.

### Brief Description of the Drawings

FIG. 1 is a perspective view schematically showing the biological electrode of one embodiment of the present invention.
FIG. 2 is an exploded perspective view of the biological electrode shown in FIG. 1.
FIG. 3 is a front view of the biological electrode shown in FIG. 1.
FIG. 4 is a side view of the biological electrode shown in FIG. 1.
FIG. 5 is a sectional view taken along line A-A in FIG. 3.
FIG. 6 is a side view showing a state in which an electrode member of the biological electrode shown in FIG. 1 is pushed in the axial direction.
FIG. 7 is an explanatory view showing an example of using a biological electrode for electroencephalogram measurement.
FIG. 8 is a side view schematically showing another example of a support shaft member.
FIG. 9 is a side view schematically showing still another example of the support shaft member.

### Mode for Carrying out the Invention

Embodiments of the present invention will be described below with reference to the drawings. It should be understood that the present invention is not limited to the following embodiments, and that appropriate modifications, changes, improvements, and the like of the design can be added based on ordinary knowledge of a skill in the art without departing from the spirit of the present invention.

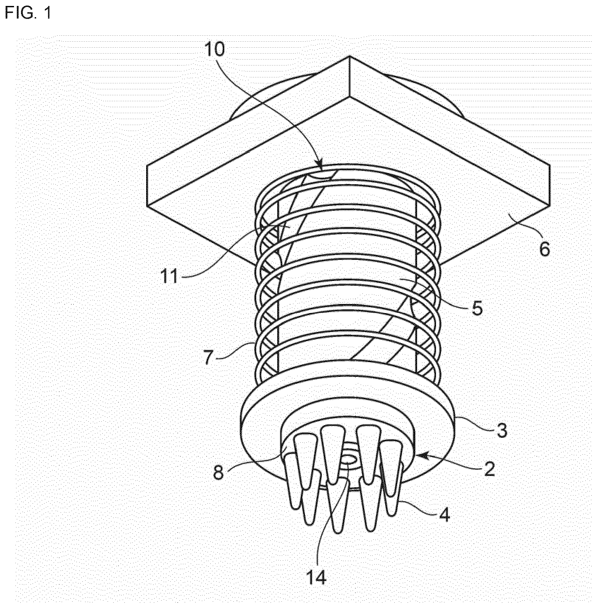

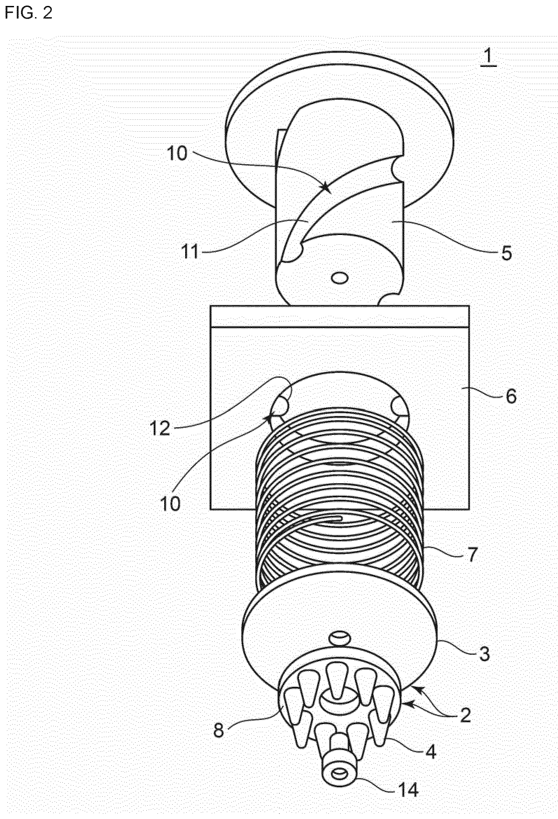

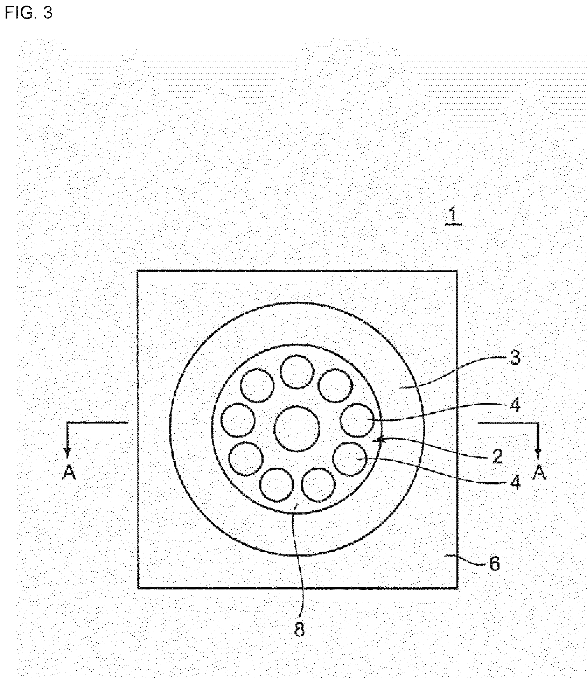

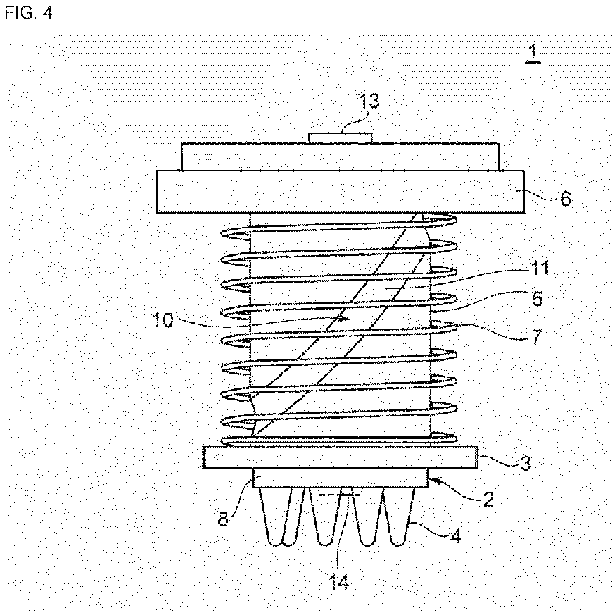

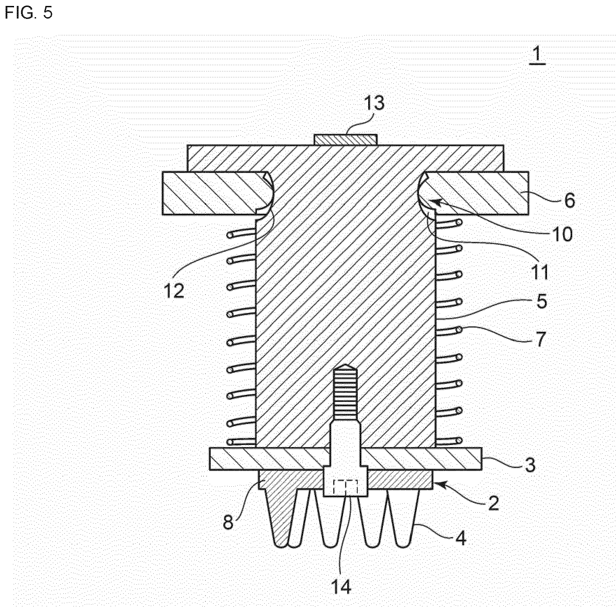

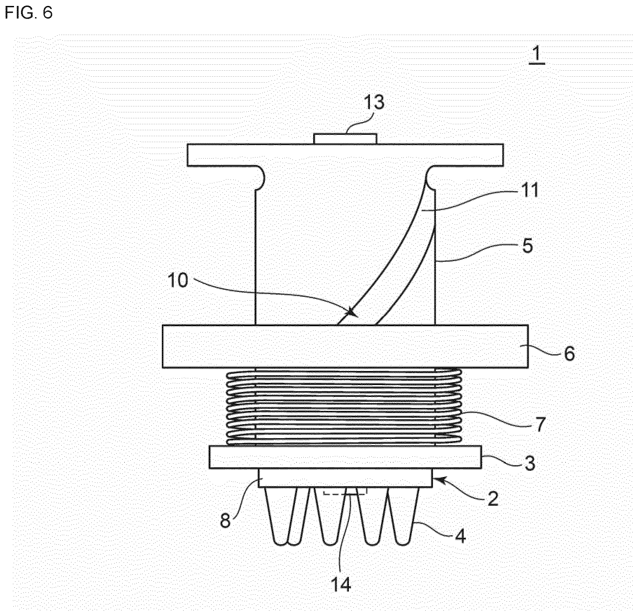

FIG. 1 is a perspective view schematically showing the biological electrode of one embodiment of the present invention. FIG. 2 is an exploded perspective view of the biological electrode shown in FIG.1. FIG. 3 is a front view of the biological electrode shown in FIG. 1. FIG. 4 is a side view of the biological electrode shown in FIG. 1. FIG. 5 is a sectional view taken along line A-A in FIG. 3. FIG. 6 is a side view showing a state in which the electrode member of the biological electrode shown in FIG. 1 is pushed in an axial direction thereof.

The biological electrode 1 shown in FIGS. 1 to 6 includes an electrode member 2, a conductive support shaft member 5 for supporting the electrode member 2, a frame member 6 for slidably holding the support shaft member 5 in an axial direction thereof, and an elastic member 7 for biasing the electrode member 2 toward the outside in the axial direction of the support shaft member 5. The "axial direction" of the support shaft member 5 means a direction in which the pillar-shaped support shaft member 5 extends from one end of the support shaft member 5 that supports the electrode member 2.

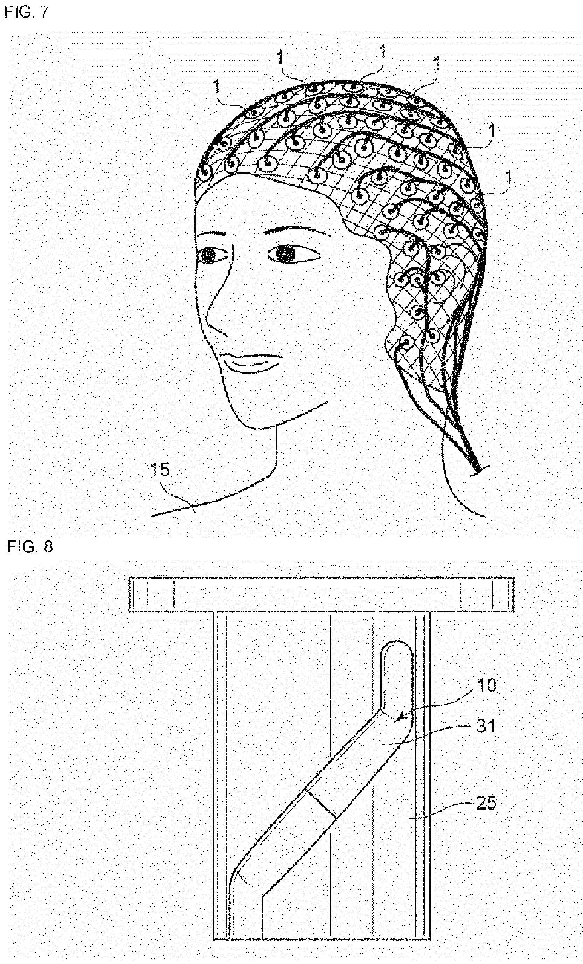

The biological electrode 1 can be suitably used for contacting the electrode member 2 with a body of a subject for detection to sense an electrical signal from the body of the subject, transmit electrical stimulation to the subject, or both. Specifically, for example, it can be used as a biological electrode 1 for a medical measuring instrument, a wearable measuring instrument, a health monitoring instrument, and the like. In particular, the biological electrode 1 can be suitably used when measuring an electroencephalogram as an electric signal. For example, as shown in FIG. 7, a plurality of biological electrodes 1 can be arranged on the scalp of the subject 15 for detection, and electroencephalogram measurement of the subject 15 can be performed. FIG. 7 is an explanatory view showing an example in which a biological electrode is used for electroencephalogram measurement.

As shown in FIGS. 1 to 6, the electrode member 2 includes a plate-shaped electrode body portion 3, and a plurality of electrode protrusions 4 provided so as to protrude in a brush shape from the electrode body portion 3. Each of the plurality of electrode protrusions 4 is electrically connected to the plate-shaped electrode body portion 3, and various electric signals are transmitted from each of the electrode protrusions 4 to the electrode body portion 3 by bringing the tip portion of the electrode protrusion 4 into contact with the body of the subject 15 for detection (see FIG. 7). The plurality of electrode protrusions 4 may have a structure integrated by the common base portion 8. With this configuration, the plurality of brush-shaped electrode protrusions 4 can be collectively attached to the electrode body portion 3 via the base portion 8. Therefore, manufacturing of the biological electrode 1 can be conveniently performed. Further, it is possible to improve the work efficiency at the time of replacement of the electrode member 2.

Support shaft member 5 is a conductive support for supporting the electrode member 2. For example, the electrode member 2 is fixed to one end side of the support shaft member 5 by a fixing screw 14 or the like. Electrical signal detected by the electrode member 2 is transmitted to the terminal portion 13 disposed on the other end side via the conductive support shaft member 5. A method of fixing the electrode member 2 into the support shaft member 5 is not limited to the method by the fixing screw 14. As the method of fixing the electrode member 2 into the support shaft member 5, for example, a method of fixing by bonding using a conductive adhesive or the like can be employed. However, it is preferable that the electrode member 2 is detachable to the support shaft member 5. By making the electrode member 2 detachable, it is possible to improve the work efficiency at the time of replacement of the electrode member 2.

The frame member 6 is for slidably holding the support shaft member 5 in an axial direction thereof, and may be configured by, for example, a part of head mounting member for mounting on the head of the subject 15 for detection. The frame member 6 has a holding space corresponding to the outer diameter of the support shaft member 5, and the support shaft member 5 is inserted into the holding space and slidably held. The frame member 6 is preferably made of an electrically insulating material, and may be configured by a part of the outer covering of the biological electrode 1.

The support shaft member 5 and the frame member 6 of the biological electrode 1 have a rotation guide mechanism 10 for converting a part of a pressing force for pushing the electrode member 2 in the axial direction of the support shaft member 5 into a rotational force in which the support shaft member 5 is a rotation axis thereof. For example, as a rotation guide mechanism 10 for rotating the electrode member 2, the support shaft member 5 is provided with a spiral groove portion 11 on its outer peripheral surface, and the frame member 6 is provided with a fitting protrusion 12 for fitting the groove portion 11 in the inner peripheral surface which holds the support shaft member 5. Therefore, the support shaft member 5 held by the frame member 6 include a rotational movement along the spiral groove portion 11 in the sliding movement in which the fitting protrusion 12 of the frame member 6 becomes a guide. Incidentally, "spiral groove portion 11" is a groove portion 11 provided on the outer peripheral surface of the support shaft member 5, and means a groove portion 11 provided so as to circle around the outer peripheral surface of the support shaft member 5 along the axial direction of the support shaft member 5.

In the biological electrode 1, the electrode member 2 is pressed in an axial direction thereof against the biasing force of the elastic member 7, so that the electrode member 2 is pushed in the axial direction while being rotated in the peripheral direction of the support shaft member 5 by the action of the rotation guide mechanism 10 described above (For example, see FIG. 6). In addition, when the pressing force pushing the electrode member 2 is released or is smaller than the biasing force of the elastic member 7, the electrode member 2 receives the bias from the elastic member 7 and is pushed out in the axial direction while rotating in the opposite direction to the pushing, and returns. Such a biological electrode 1 can realize good contact with the skin of the subject 15 for detection (see FIG. 7). In particular, the biological electrode 1 is suitable for measurement of an electrical signal at a part having body hair in the body of the subject 15 (see FIG. 7), and particularly suitable for electroencephalogram measurement. For example, in electroencephalogram measurement, it is possible to positively push hair aside when mounting the biological electrode 1 to realize extremely good contact with the scalp of the subject 15 (see FIG. 7). That is, the electrode member 2 is rotated in the peripheral direction of the support shaft member 5, whereby a plurality of electrode protrusions 4 provided on the electrode member 2 rotate on the scalp surface of the subject 15 (see FIG. 7) so as to circle around the rotation axis of the electrode member 2. Therefore, when the electrode protrusion 4 of the electrode member 2 is brought into contact with the scalp of the subject 15 (see FIG. 7), even if the hair or the like is caught between the electrode protrusion 4 and the scalp, the rotational movement of the electrode protrusion 4 extrude the hairs caught between the electrode protrusion 4 and the scalp to the periphery, and it is possible to positively push the hair aside. Further, as the electrode member 2 return to the original position by biasing from the elastic member 7, each time a pressing force is applied to the electrode member 2, the rotational movement of the electrode protrusions 4 described above is repeatedly performed. Therefore, the biological electrode 1 can improve the contact state with the subject 15 for detection (see FIG. 7) by applying the pressing force to the electrode member 2 without requiring time and labor such as remounting the once placed biological electrode 1. In addition, since the electrode member 2 is constantly biased toward the subject 15 (see FIG. 7) side by the elastic member 7, the electrode member 2 does not fluctuate in the axial direction of the support shaft member 5, thereby realizing reliable contact with the subject 15. Further, even in a case where there is variation in size due to an individual difference in the subject 15 (see FIG. 7) and unevenness on the skin surface, insufficient contact of the electrode member 2 hardly occurs because the electrode member 2 is biased by the elastic member 7, and a good contact state can be realized.

The biological electrode 1 is suitable for measuring an electric signal at a part having body hair as described above, however, it does not hinder a use at a part not having body hair. Further, the biological electrode 1 can be used for a creature other than a human. For example, a creature (so-called an animal or the like) having body hair such as a mammal like a dog or a cat may be used as a subject for detection, and various electric signals of the body thereof can be measured by using the biological electrode 1 as an electrode.

Pressing force pushing the electrode member 2 may be any force to resist the biasing force of the elastic member 7. For example, when the biological electrode 1 is provided on the head mounting member such as a head gear, the electrode member 2 is pressed in the axial direction so as to resist the biasing force of the elastic member 7 by the tightening pressure when the head gear is mounted on the head of the subject 15 (see FIG. 7). In addition, after the electrode member 2 is brought into contact with the scalp of the subject 15 (see FIG. 7), a pressing force may be applied to the electrode member 2 by lightly pushing the biological electrode 1 to confirm contact with the scalp. For example, in the case where the frame member 6 is connected and fixed to a head mounting member for mounting on the head of the subject 15 (see FIG. 7), the rotational movement of the electrode member 2 can be promoted by, for example, pressing the entire head mounting member or a part thereof toward the subject 15 (see FIG. 7) side. It is to be noted that, at the time of measuring a biological signal such as an electroencephalogram, it is more preferable that the electrode member 2 is pushed by a force that resists the biasing force of the elastic member 7 with respect to the body side of the subject. With this configuration, even when vibration or the like is applied to the electrode member 2, the contact point of the electrode member 2 is hardly shifted, and the mixing of noise components or the like can be effectively suppressed.

In spiral groove portion 11 provided on the support shaft member 5 as the rotation guide mechanism 10, the number of circling in the outer peripheral surface of the support shaft member 5 may be one or more, the number of circling in the outer peripheral surface may be less than one. Depending on the axial length of the support shaft member 5, it is possible to appropriately determine such circling amount and spiral pitch of the groove portion 11 in the outer peripheral surface of the support shaft member 5. The spiral pitch refers to the length of one circling of the spiral groove portion 11. With respect to the peripheral length of the outer peripheral surface of the support shaft member 5, when the spiral pitch is increased, the rotation amount of the support shaft member 5 and the electrode member 2 according to the sliding amount in the axial direction of the support shaft member 5 is reduced.

The rotation guide mechanism 10 may be any one that converts a part of the pressing force that pushes the electrode member 2 in the axial direction thereof into a rotating force with the support shaft member 5 as a rotating shaft thereof, and is not limited to the spiral groove portion 11 of the support shaft member 5 and the fitting protrusion 12 of the frame member 6 as shown in FIGS. 1 to 6. For example, although not shown, a spiral fitting protrusion may be provided on the outer peripheral surface of the support shaft member 5, and a groove portion for fitting with the spiral fitting protrusion may be provided on the inner peripheral surface of the frame member 6.

As another example of the support shaft member, a support shaft member 25 as shown in FIG. 8 can be cited. FIG. 8 is a side view schematically showing another example of the support shaft member. The support shaft member 25 is provided with the groove portion 31 on the outer peripheral surface thereof as a rotation guide mechanism for applying a rotational force to the support shaft member 25. In the one end side and the other end side of the support shaft member 25, the groove portion 31 is formed in a linear shape along the axial direction of the support shaft member 25. In the intermediate portion of the support shaft member 25, the groove portion 31 is formed so as to extend obliquely with respect to the axial direction of the support shaft member 25 so as to circle the outer peripheral surface of the support shaft member 25. In the case that the support shaft member 25 as shown in FIG. 8 is used for the biological electrode 1 as shown in FIGS. 1 to 6, when the electrode member 2 is pressed in the axial direction thereof so as to resist the biasing force of the elastic member 7, immediately after pressing, the electrode member 2 is pushed straight in the axial direction without rotating, and after a constant pushing is made, rotation of the electrode member 2 is started. Such a biological electrode 1 can also realize good contact with the skin of the subject 15 (see FIG. 7).

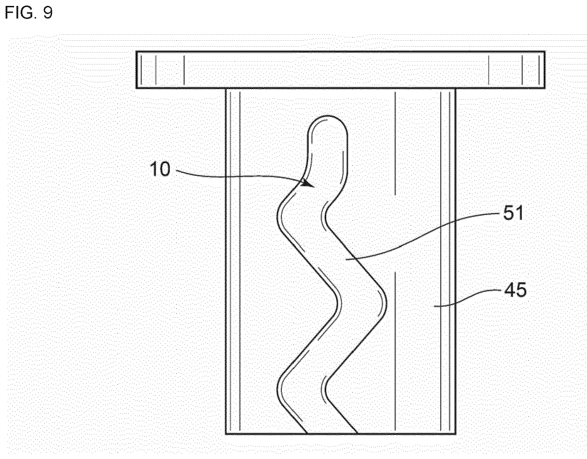

As still another example of the support shaft member, a support shaft member 45 as shown in FIG. 9 can be cited. FIG. 9 is a side view schematically showing still another example of the support shaft member. The support shaft member 45 is provided with the wavy groove portion 51 extending from one end side to the other end side on its outer peripheral surface as a rotation guide mechanism for applying a rotational force to the support shaft member 45. In the case that the support shaft member 45 as shown in FIG. 9 is used for the biological electrode 1 as shown in FIGS. 1 to 6, when the electrode member 2 is pressed in the axial direction thereof so as to resist the biasing force of the elastic member 7, in the middle of pressing the electrode member 2, the rotation direction of the electrode member 2 is changed. That is, by pushing the electrode member 2 in the axial direction thereof, the electrode member 2 rotates by a certain angle along the shape of the wavy groove portion 51 (hereinafter, this rotation direction is referred to as "forward rotation") and thereafter, the electrode member 2 rotates in the reverse direction with the apex of the waveform as a boundary. Thereafter, each time passing through each apex of the waveform, so that the electrode member 2 repeats the forward rotation and reverse rotation. By the waveform of the groove portion 51 provided on the outer peripheral surface of the support shaft member 45, it is possible to realize two types of rotation of forward rotation and reverse rotation by the pressing once of the electrode member 2.

As shown in FIGS. 1 to 7, in the biological electrode 1, a plurality of electrode protrusions 4 constituting the electrode member 2 are in direct contact with the skin of the subject 15 to transfer an electric signal to and from the skin. The electrode member 2 is preferably made of a conductive material, for example, metal, conductive rubber, or the like. Examples of the metal include stainless steel, copper, and aluminum. Examples of the conductive rubber include conductive rubber containing conductive carbon particles, silver powder, or flake-like silver particles. Examples of the rubber component in the conductive rubber include silicone rubber.

The plurality of electrode protrusions 4 are used for transmitting and receiving an electric signal as described above, and also serve as a brush for pushing aside the hair of the subject 15 in the biological electrode 1. There is no particular limitation on the number of electrode protrusions 4 in one electrode member 2, and the optimum number can be appropriately determined according to the size of the electrode member 2 (in other words, the area of the electrode member 2 covering the skin of the subject 15), the measurement site of the body of the subject 15, the type of the electric signal, and the like. For example, in the example shown in FIG. 3, nine electrode protrusions 4 are arranged around the rotation axis of the electrode member 2 at 40° intervals with respect to one electrode member 2, however, the number of the electrode protrusions 4 is not limited to nine. In addition, a plurality of electrode protrusions 4 may be arranged so as to concentrically surround the circumference of the electrode member 2 in double or triple around the rotation axis of the electrode member 2. The plurality of electrode protrusions 4 may be arranged at regular intervals (equal intervals) or irregularly.

The shape of the electrode protrusion 4 is not particularly limited as long as it can come into good contact with the body of the subject 15. For example, it is preferable that the electrode protrusion 4 has a rod shape with a bullet-shaped tip. By protruding a plurality of electrode protrusions 4 having such a shape in a brush shape, the tip of the electrode protrusion 4 easily comes into contact with the scalp by slipping through the hair or the like. Further, since the tip end of each electrode protrusion 4 has a rounded shape, it is in soft contact with the scalp, so that discomfort is not caused to the subject 15. The shape of each of the electrode protrusions 4 is not particularly limited as described above, and is not limited to the illustrated shape, and can be appropriately determined according to the measurement site of the body of the subject 15, the type of the electric signal, and the like.

The protrusion length and the thickness of the electrode protrusion 4 are not particularly limited. For example, the protrusion length and the thickness are appropriately set so that the electrode protrusion 4 can be brought into contact with the scalp by slipping through the hair, and can effectively push the hair aside by the rotation of the electrode member 2. For example, for a subject 15 having a relatively large amount of hair, the protrusion length of the electrode protrusion 4 may be increased. In addition, for a subject 15 having a relatively small amount of hair or a young subject 15 such as an infant, the protrusion length of the electrode protrusion 4 may be shortened or the thickness of the electrode protrusion 4 may be reduced. The specific protrusion length of the electrode protrusion 4 may be, for example, 6 to 15mm. For example, when the protrusion length of the electrode protrusion 4 is extremely short, there is a tendency that a space for storing hair is reduced, and the electrode member 2 tends to float easily due to the hair and is difficult to come into contact with the skin.

The common base portion 8 for integrating the plurality of electrode protrusions 4 is preferably integrally formed of the same conductive material as that of the electrode protrusions 4. Although there is no particular limitation on the shape of the base portion 8, it can be formed in the same plate shape as the electrode portion 3, for example, in a disc shape.

The electrode body portion 3 is a plate-like member for connecting and fixing the electrode member 2 to the support shaft member 5. The electrode body portion 3 is also used to apply the biasing force of the elastic member 7 to the electrode member 2. The electrode body portion 3 is preferably made of a conductive material, for example, metal, conductive rubber, or the like, in order to satisfactorily transmit the electric signal transmitted and received by the plurality of electrode protrusions 4 to the support shaft member 5. The electrode protrusion 4 and the electrode body portion 3 may be formed of the same material or different materials.

The support shaft member 5, as well as supporting the electrode member 2, has a rotation guide mechanism 10 for converting the pressing force to the rotational force with respect to the electrode member 2. The support shaft member 5 is preferably made of a conductive material, for example, metal, conductive rubber, or the like. The support shaft member 5 may not be made of a conductive material as a whole as long as the support shaft member 5 can ensure electrical connection between the electrode member 2 disposed on one end side in the axial direction and the terminal portion 13 disposed on the other end side. For example, a conductive portion made of a conductive material is disposed in the central portion in the axial direction of the support shaft member 5, and an outer peripheral portion made of a conductive material or a non-conductive material may be provided so as to cover the conductive portion. In such a support shaft member 5, the electrical connection of the electrode member 2 and the terminal portion 13 is ensured by the conductive portion of the central portion in the axial direction. The outer peripheral portion of the support shaft member 5 may be used appropriately optimum material in consideration of such workability and durability of the spiral groove portion 11.

The terminal portion 13 provided on the other end side of the support shaft member 5 may be formed integrally with the support shaft member 5, or may be formed separately from the support shaft member 5. If the terminal portion 13 is formed separately from the support shaft member 5, the terminal portion 13 can be formed by a material suitable for electrical connection with the connection wiring. For example, the terminal portion 13 may be made of metal, and may be adhered to the surface of the other end side of the support shaft member 5 by a conductive adhesive, or may be embedded in the support shaft member 5 so that a part thereof protrudes from the surface.

The frame member 6 is for holding the support shaft member 5 slidably in the axial direction of the support shaft member 5, and may be configured by, for example, a part of a mounting member for mounting the biological electrode 1 to the body of the subject 15, or may be configured to be detachable from the mounting member. It is preferable that the frame member 6 is fixed to the above-described mounting member in a state of being non-rotatable in the peripheral direction of the support shaft member 5 when the biological electrode 1 is mounted on the body of the subject 15. With this configuration, the electrode member 2 and the support shaft member 5 are preferentially rotated by the operation of pushing the electrode member 2, and the contact state with the subject 15 can be effectively improved. Examples of the mounting member for mounting the biological electrode 1 to the body of the subject 15 include a head mounting member such as a headgear for mounting on the head and an electrode cap for electroencephalogram formed in a net shape.

The frame member 6 is preferably made of an electrically insulating material in order to suppress the mixing of noise components other than the electrical signal transmitted and received by the electrode member 2 and biological signals other than the object.

The outer shape of the frame member 6 is not particularly limited as long as it can hold the support shaft member 5 slidably by a holding space corresponding to the outer diameter of the support shaft member 5. For example, in FIGS. 1 to 6, the frame member 6 in which the holding space corresponding to the outer diameter of the support shaft member 5 is formed in a rectangular frame body is shown, however, the outer peripheral shape of the frame body is not limited to a rectangular shape or the like.

The elastic member 7 is for applying a biasing force to the electrode member 2. Therefore, even if the electrode member 2 and the support shaft member 5 are configured to be slidable in the axial direction thereof, the electrode member 2 does not fluctuate in the axial direction of the support shaft member 5, and the electrode member 2 can realize reliable contact with the subject 15. Further, by having such an elastic member 7, when the pressing force pushing the electrode member 2 is released, it is possible to return the electrode member 2 to the original position in the axial direction thereof. Therefore, the electrode member 2 can repeatedly rotate by pushing the electrode member 2 returned to the original position in the axial direction again in the axial direction while the biological electrode 1 is mounted.

The elastic member 7 may be, for example, a coil spring provided so as to wind the outer periphery of the support shaft member 5. However, the elastic member 7 may be made of an elastic body other than a coil spring as long as it applies a biasing force to the electrode member 2. Examples of the other elastic body include leaf springs, coned disc springs, and the like. Further, the elastic member 7 may be a rubber or a foam having resilience. For example, the elastic member is configured to have a cylindrical shape by rubber or the like, and the cylindrical elastic member may be disposed on the outer periphery of the support shaft member 5.

The magnitude of the biasing force acting on the electrode member 2 of the elastic member 7 is not particularly limited. If the biasing force of the elastic member 7 is too large, when the electrode member 2 is pushed in the axial direction thereof, the tip of the electrode protrusion 4 bites into the skin of the subject 15, which may cause pain in the subject 15. The biasing force of the elastic member 7 is preferably such a magnitude as not to cause pain to the subject 15 when the electrode member 2 is pushed in the axial direction thereof. When the frame member 6 is composed of, for example, a part of the head mounting member, a constant pressure is applied to the electrode member 2 by tightening the head by the head mounting member when the biological electrode 1 is mounted. At this time, if the biasing force of the elastic member 7 is smaller than the pressure at the time of mounting the biological electrode 1, the electrode member 2 may be completely pushed in the axial direction thereof at the time of mounting the biological electrode 1. Of course, by tightening the head when the biological electrode 1 is mounted, a rotational movement of the electrode member 2 occurs, so that a certain effect of pushing the hair of the subject 15 aside can be obtained. However, in order to perform the rotational movement of the electrode member 2 even after the biological electrode 1 is mounted, the biasing force of the elastic member 7 is preferably larger than the pressure at the time of mounting the biological electrode 1.

### Industrial Applicability

The biological electrode of the present invention can be utilized as a biological electrode for contacting a body of the subject to sense electrical signals from the body of the subject, transmit electrical stimulation to the subject, or both sensing and transmitting as described above.

### Description of Reference Numerals

- 1:: Biological electrode
- 2:: Electrode member
- 3:: Electrode body portion
- 4:: Electrode protrusion
- 5,25,45:: Support shaft member
- 6:: Frame member
- 7:: Elastic member
- 8:: Base portion
- 10:: Rotation guide mechanism
- 11,31,51:: Groove portion
- 12:: Fitting protrusion
- 13:: Terminal portion
- 14:: Fixing screw
- 15:: Subject for detection

## Claims

1. A biological electrode comprising,
an electrode member in contact with a body of a subject for detection,
a conductive support shaft member for supporting the electrode member,
a frame member for slidably holding the support shaft member in an axial direction thereof, and
an elastic member for biasing the electrode member toward the outside in the axial direction of the support shaft member,
wherein the electrode member includes a plate-shaped electrode body portion and a plurality of electrode protrusions provided so as to protrude in a brush shape from the electrode body portion, and
wherein the support shaft member and the frame member have a rotation guide mechanism that converts a part of a pressing force for pushing the electrode member in the axial direction of the support shaft member into a rotational force in which the support shaft member is a rotation axis thereof.

2. The biological electrode according to claim 1, wherein the rotation guide mechanism includes a spiral groove portion provided on an outer peripheral surface of the support shaft member, and a fitting protrusion fitted to the groove portion and provided on an inner peripheral surface of the frame member for holding the support shaft member.

3. The biological electrode according to claim 1 or 2, wherein the electrode protrusion is made of conductive rubber.

4. The biological electrode according to any one of claims 1 to 3, wherein the elastic member is a coil spring provided so as to wind the outer periphery of the support shaft member.

5. The biological electrode according to any one of claims 1 to 4, which is used for electroencephalogram measurement of the subject.

6. The biological electrode according to claim 5, wherein the frame member is part of a head mounting member for mounting to a head of the subject.

7. The biological electrode according to any one of claims 1 to 6, further comprising a terminal portion for transmitting and receiving an electric signal from the electrode member at an end of the support shaft member opposite to the side on which the electrode member is disposed.
